# EUROPÄISCHE PATENTSCHRIFT

(11) **EP 2 233 150 B1**
(45) Veröffentlichungstag und Bekanntmachung des Hinweises auf die Patenterteilung: **07.09.2016**
(21) Anmeldenummer: 10005293.5
(22) Anmeldetag: 30.12.2004
(51) Int. Cl.: A61K 38/18, A61K 35/407, A61K 35/12, A61P 17/02, A61P 1/16

(54) **Erythropoietin zur Anwendung bei der Behandlung von Wunden oder der Transplantation von Zellen**
Erythropoietin for use in the treatment of wounds or the transplantation of cells
Erythropoietin pour utilisation dans la guérison de blessures ou la transplantation de cellules

(30) Priorität: 30.12.2003 DE 10361813; 30.12.2003 EP 03029961
(43) Veröffentlichungstag der Anmeldung: 29.09.2010
(62) Teilanmeldung aus: 04804424.2
(73) Patentinhaber: Bader, Augustinus, 04668 Parthenstein / OT Klinga (DE)
(72) Erfinder: Bader, Augustinus, 04668 Parthenstein / OT Klinga (DE)
(74) Vertreter: Benz, Jürgen

(56) Entgegenhaltungen:
- WO-A2-2004/001023
- US-B1- 6 660 905
- HAROON ZISHAN A ET AL: "A novel role for erythropoietin during fibrin-induced wound-healing response.", AMERICAN JOURNAL OF PATHOLOGY, Bd. 163, Nr. 3, September 2003 (2003-09), Seiten 993-1000, XP002674608, ISSN: 0002-9440
- BUEMI MICHELE ET AL: "Recombinant human erythropoietin influences revascularization and healing in a rat model of random ischaemic flaps.", ACTA DERMATO-VENEREOLOGICA 2002 LNKD- PUBMED:12575845, Bd. 82, Nr. 6, 2002, Seiten 411-417, XP002993404, ISSN: 0001-5555
- GALEANO M ET AL: "Recombinant human erythropoietin stimulates angiogenesis and wound healing in the genetically diabetic mouse", DIABETES, AMERICAN DIABETES ASSOCIATION, US, Bd. 53, Nr. 9, 1. September 2004 (2004-09-01), Seiten 2509-2517, XP002637869, ISSN: 0012-1797
- ROCHE R J: "Does human recombinant erythropoietin improve wound healing?", JOURNAL OF THE AMERICAN GERIATRICS SOCIETY JAN 1995 LNKD- PUBMED:7806748, Bd. 43, Nr. 1, Januar 1995 (1995-01), Seite 81, XP009157788, ISSN: 0002-8614
- WILLIAMSON ET AL: "Erythropoietin", TRANSFUSION SCIENCE, PERGAMON PRESS, OXFORD, GB, Bd. 12, Nr. 1-2, 1. Januar 1991 (1991-01-01), Seiten 15-23, XP022645740, ISSN: 0955-3886, DOI: 10.1016/0955-3886(91)90005-N [gefunden am 1991-01-01]
- MOGHTADER ET AL: "The use of recombinant human erythropoietin and cultured epithelial autografts in a Jehovah's witness with a major thermal injury", BURNS, BUTTERWORTH HEINEMANN, GB, Bd. 20, Nr. 2, 1. April 1994 (1994-04-01), Seiten 176-177, XP022220358, ISSN: 0305-4179, DOI: 10.1016/S0305-4179(06)80019-7
- FATOUROS M ET AL: "Alterations in body weight, breaking strength, and wound healing in Wistar rats treated pre- and postoperatively with erythropoietin or granulocyte macrophage-colony stimulating factor: evidence of a previously unknown anabolic effect of erythropoietin?", THE JOURNAL OF LABORATORY AND CLINICAL MEDICINE MAR 1999, Bd. 133, Nr. 3, März 1999 (1999-03), Seiten 253-259, XP009158639, ISSN: 0022-2143
- DEMPKE W ET AL: "HUMAN HEMATOPOIETIC GROWTH FACTORS: OLD LESSONS AND NEW PERSPECTIVES", ANTICANCER RESEARCH, INTERNATIONAL INSTITUTE OF ANTICANCER RESEARCH, GR, Bd. 20, Nr. 6D, 5. Dezember 1999 (1999-12-05), Seiten 5155-5164, XP009005227, ISSN: 0250-7005
- MILLER C B ET AL: "Erythropoietin in stem cell transplantation.", BONE MARROW TRANSPLANTATION MAY 2001, Bd. 27, Nr. 10, Mai 2001 (2001-05), Seiten 1011-1016, XP002687666, ISSN: 0268-3369
- BARON FRÉDÉRIC ET AL: "Optimization of recombinant human erythropoietin therapy after allogeneic hematopoietic stem cell transplantation", EXPERIMENTAL HEMATOLOGY, ELSEVIER INC, US, Bd. 30, Nr. 6, 1. Juni 2002 (2002-06-01), Seiten 546-554, XP009157801, ISSN: 0301-472X, DOI: 10.1016/S0301-472X(02)00795-6 [gefunden am 2002-06-05]
- DATABASE MEDLINE [Online] US NATIONAL LIBRARY OF MEDICINE (NLM), BETHESDA, MD, US; September 2000 (2000-09), SUGIYAMA N ET AL: "Intraportal administration of low-dose recombinant human hepatocyte growth factor enhances effects of hepatocellular transplantation.", XP002687836, Database accession no. NLM11100324 & SUGIYAMA N ET AL: "Intraportal administration of low-dose recombinant human hepatocyte growth factor enhances effects of hepatocellular transplantation.", HEPATO-GASTROENTEROLOGY 2000 SEP-OCT, Bd. 47, Nr. 35, September 2000 (2000-09), Seiten 1245-1249, ISSN: 0172-6390
- LECKEL K ET AL: "[State of hepatocyte transplantation: a risk or a chance?].", ZENTRALBLATT FÜR CHIRURGIE APR 2003, Bd. 128, Nr. 4, April 2003 (2003-04), Seiten 283-290, XP009165041, ISSN: 0044-409X
- BILIR B M ET AL: "Hepatocyte transplantation in acute liver failure", LIVER TRANSPLANTATION, SAUNDERS, PHILADELPHIA, PA, US, Bd. 6, Nr. 1, 1. Januar 2000 (2000-01-01), Seiten 32-40, XP026696479, ISSN: 1527-6465 [gefunden am 2000-01-01]

## Beschreibung

Diese Anmeldung ist eine Teilanmeldung des Patentes EP 1 699 915 B1 und betrifft ein Verfahren zur Induktion des strukturellen Wachstums von Gewebe mit Hilfe von Wachstumsfaktoren, insbesondere Erythropoeitin (EPO).

In der Ontogenese werden Wachstumsfaktoren exprimiert, die grundlegende strukturelle und hinsichtlich der Zellzahl numerische Prozesse für den Aufbau eines Gewebes auslösen können. Im wachsenden und im adulten Organismus geht die Fähigkeit, strukturell und funktionell intakte Gewebe aufzubauen oder- im Falle von Gewebeschädigungen - zu regenerieren, allerdings weitgehend verloren. Es wird vermutet, dass die verminderte Expression von Wachstumsfaktoren, die ihrerseits die Expression von für den Gewebeaufbau erforderlichen Proteinen kontrollieren, für diese Reduktion an Regenerationsfähigkeit verantwortlich ist.

Der Ersatz der Organmasse durch Zellproliferation allein stellt daher keine ausreichende Basis für die Therapie eines Patienten mit erheblichem Organschaden dar. Es sind daher verschiedene Ansätze für die Induktion strukturellen Wachstums - d.h. für ein formschaffendes Wachstum - von Geweben bekannt, die jedoch allesamt noch nicht zu einem befriedigenden Erfolg geführt haben. Ein solches strukturelles Wachstum von Zellen wäre jedoch gerade für therapeutische oder biotechnologische Verfahren von erheblicher Bedeutung.

Jüngere Veröffentlichungen deuten an, dass an sich hämatopoietische Cytokine wie Erythropoietin (EPO) bei der Regeneration von Gewebe wirksam sein können.

So berichten Haaron et al. (2003, Am. J. Pathol., 163(3), S. 993 -1000) über eine neue Wirkung von Erythropoietin (EPO). Demgemäß wird die wundheilende Wirkung von EPO bei Tierversuchen, nämlich bei Fibrin-induzierter Wundheilung (fibrin Z-chamber) offenbart, wonach die lokale und exogene Gabe von EPO, injiziert in eine mit Fibrin gefüllte Kammer, zu einer signifikanten Bildung von Granulationsgewebe mit erhöhter Angiogenese führt.

Buemi et al. (2002, Acta Dermato-Venereologica, 82 (6) S. 411-417) offenbaren die Fähigkeit von EPO, Wundheilungsprozesse zu beschleunigen, und zeigen, dass die subkutane Gabevon EPO bei Ratten mit ischämischen Hautlappen zu einer rascheren Bildung von Granulationsgewebe sowie zu einer erhöhten Revaskularisierung der ischämischen Hautlappen führt.

Die Aussagen in den beiden genannten Dokumenten beruhen auf Ergebnissen aus Versuchen, welche mehr oder weniger unter artifiziellen Bedingungen durchgeführt wurden. Beide Autorengruppen offenbaren jedoch nicht, dass die so beobachtete Bildung von Granulationsgewebe unter realen in vivo-Bedingungen einer Verletzung bei EPO-Gabe auch einen Gewebestruktur bildenden Effekt ausüben könnte, welcher letztlich zu einer gewünschten narbenfreien (ohne Granulationsgewebe) Wundheilung beitragen würde.

Aufgabe der vorliegenden Erfindung ist es daher, ein Verfahren bereitzustellen, das die Induktion eines im Wesentlichen strukturellen Wachstums eines Gewebes induziert. Dieses Wachstum sollte vorzugsweise zu einer im Wesentlichen funktionellen und strukturellen Funktionsfähigkeit des betroffenen Gewebes führen, insbesondere bei der Wundheilung im Zusammenhang mit Verbrennungen und Verbrühungen.

Es hat sich überraschenderweise gezeigt, dass durch die Applikation von hämatopoietischen Wachstumsfaktoren wie EPO nicht nur eine Vermehrung der Zellen, sondern auch ein strukturelles Wachstum einsetzt. Dieses Wachstum setzt insbesondere an zuvor traumatisierten Geweben ein. Das dadurch induzierte Wachstum führt *in vivo* zu einer Gewebe-Regeneration im eigentlichen Sinne, d.h. es findet nicht nur proliferatives Wachstum, sondern gerichtetes, differenziertes Wachstum zum Aufbau komplexer Strukturen statt.

Die Erfindung betrifft Erythropoietin (EPO) zur Anwendung bei der Wundheilung ohne Narbenbildung nach Brandverletzungen unter Verwendung eines Hauttransplantats.

Die Erfindung betrifft außerdem Erythropoietin (EPO) zur systemischen oder topischen Anwendung bei der Regeneration von durch Verbrennungen oder Verbrühungen traumatisiertem Gewebe unter Verwendung eins Transplantates, Implantates oder eines Trägermaterials.

Im Kern basiert die Verwendung der hämatopoietischen Faktoren für die Geweberegeneration im Wesentlichen auf zwei bisher nicht bekannten Wirkungen des EPO, nämlich einerseits auf der Stimulation des strukturellen Wachstums in synchroner und koordinierter Form von verschiedener Zelltypen untereinander und miteinander (wie z.B. Fibroblasten, glatten Muskelzellen mit Endothelzellen im Gefäßbereich in Verbindung mit einer Neubildung der Architektur eines kompletten Gefäßes unter Berücksichtigung der extrazellulären Matrix (Kollagen, Elastin, Fibronektin, Entaktin)) und einer Komplettierung des eigentlichen parenchymatösen Gewebeverbandes. Neben der Ausbildung eines tatsächlichen Gefäßbaumes und dessen Interkonnektion wird somit erfindungsgemäß eine Geweberegeneration im Sinne der *restitutio ad integrum* induziert.

Wie bereits ausgeführt, setzt die Wirkung der hämatopoietischen Wachstumsfaktoren insbesondere bei traumatisierten Geweben und Zellen ein. Dabei wird der Begriff des Traumas als Gegensatz zu dem Prozess der Histogenese (Gewebebildung) definiert. Es handelt sich demnach bei einem Trauma um einen Prozess, der der Histogenese als Gewebildungsprozess im Individualorgismus an den betreffenden Lokalisationen entgegenwirkt bzw. das Ergebnis der Histogenese zunichtemacht. Das Trauma als Gewebeschädigung kann durch eine Vielzahl von Ereignissen ausgelöst werden, z.B. durch Verletzungen, Entzündungen oder durch Autoimmunerkrankungen mit Selbstbeschädigung). Diese Gewebeschädigungen oder -zerstörungen lösen wiederum eine Vielzahl von Reaktionen aus, so z.B. die Aktivierung von Makrophagen, Mastzellen und immunkompetenten Zellen, die chemotaktische, vasoaktive und wundheilungsfördernde Faktoren sezernieren, und dadurch systemische und regioselektive Mechanismen regulieren.

Die Vorteile der Verwendung der hämatopoietischen Wachstumsfaktoren, insbesondere des EPO, erstrecken sich auf die Regeneration von Geweben aller vier Grundgewebearten, nämlich des Bindegewebes, des Muskelgewebes, des Epithelgewebes und des Nervengewebes. Diese Gewebe leiten sich ontogenetisch entweder aus dem Mesoderm (Bindegewebe, Muskel, Endothel (als besondere Form des Epithels)), dem Endoderm (das den Gastrointestinaltrakt auskleidende Epithel) oder dem Ektoderm (Nervengewebe) ab. In der Vergangenheit wurde gezeigt, dass der EPO-Rezeptor sowohl auf Zellen meso- als auch endodermalen Ursprungs sowie auf neuronalen Zellen exprimiert wird. In diesen Geweben führt die Verwendung von EPO zu einem ortsständigen Recruitment der gewebespezifischen Progenitorenpopulation (Stammzellen), der Migration der Zellen und der Differenzierung bzw. Transdifferenzierung der Zellen in Parenchym- und Strukturzellen. Während und vor dieser Gewebsbildung vermehren sich die Zellen durch die EPO Gabe.

Bei der Anwendung des Verfahrens kann eine erneute Vervollständigung des zuvor geschädigten Organs zu einem kompletten parenchymatösen Gewebeverband erreicht werden, Endothelzellen. Mit der weiterführenden Ausbildung eines Gefäßbaums ist somit erfindungsgemäß die Geweberegeneration als *restitutio ad integrum* möglich.

Ein besonderer Vorteil des Verfahrens liegt somit darin, dass nicht nur die Mikokapillarisation des regenerierenden Gewebes über eine Endothelaussprossung stimuliert wird, sondern auch die Parenchymregeneration und die Ausbildung der Wandstrukturen gefördert werden. Erst dies führt zu dem gewünschten Ergebnis des koordinierten dreidimensionalen Wachstums zum Aufbau eines funktionsfähigen Organs.

Somit basiert die Verwendung auf einer EPO-Wirkung, die weit über die bisher bekannte EPO-Wirkung als angiogenetischer Faktor auf die Endothelzellenvermehrung hinausgeht (Journal of Nephrology 2002 15, 97 bis 103). Da mikrovaskuläre Strukturen wie Kapillaren und Sinusoide lediglich aus Endothelzellenauskleidung bestehen und keine eigene Wandstruktur aufweisen, konnte ausgehend von der angiogenetischen Wirkung des EPO bisher jedoch lediglich darüber spekuliert werden, ob EPO auch bei der Gefäßneubildung und Wundheilung eine gewisse Bedeutung zukommen könnte (Journal of Nephrology 2002 15,97 bis 103). Eine Substantiierung dieser Spekulationen blieb bislang aber aus. Daher ist es vorliegend umso bedeutender, dass erstmals der Nachweis der Wirkung des EPO auf das synchronisierte und koordinierte Wachstum der Gefäße selbst, d.h. einschließlich der Ausbildung der Wandstrukturen und die Parenchymregeneration gelungen ist. Ein weiterer Vorteil des Verfahrens besteht darin, dass das strukturelle Wachstum nicht notwendigerweise eine vorgegebene organische oder anorganische dreidimensionale Struktur als Ausgangspunkt benötigt, sondern viel eher eine Organ(teil)struktur de *novo* schafft. Somit kann die Applikation der hämatopoietischen Wachstumsfaktoren eine deutlich beschleunigte Selbst-Regeneration eines beschädigten Gewebes induzieren, was in der klinisch-therapeutischen Anwendung von großer Bedeutung ist.

In einer besonders vorteilhaften Ausführunasform werden die besaaten hämatopoietischen Wachstumsfaktoren eingesetzt, um die Regeneration eines Gewebes zu induzieren, das traumatisch geschädigte Bereiche aufweist. In diesen Gewebeabschnitten kann damit nicht nur ein Verschluss der Wunde durch die Ausbildung eines Granulationsgewebes mit einsetzender Angiogenese angeregt, sondern die Neubildung der gewebespezifischen dreidimensionalen Struktur aus extrazellulärer Matrix z.B. aus Kollagen, Elastin, Fibronektin oder Etnaktin.

Erfindungsgemäß wird EPO verwendet, das *in vivo* die Bildung von Erythrozyten sowie die Zellteilung und Differenzierung von Erythrozyten-Vorläuferzellen stimuliert. EPO ist entweder aus Urin isolierbar oder aber rekombinant herstellbar. Die Herstellung eines rekombinanten humanen EPO ist Gegenstand der WO 86/03520. Des weiteren ist EPO Gegenstand der EP 0 148 605, EP 0 205 564, EP 0 209 539, EP 0 267 678 oder EP 0 411 678. Derivate des nativen oder rekombinanten EPO sind auch bekannt.

So ist beispielsweise ein EPO-Derivat bekannt (EP 0 640 619 B1), das zusätzliche Glykosylierungsstellen und somit einen höheren Kohlenhydratanteil mit 22 Sialsäureresten aufweist. Der Vorteil dieser Modifikation besteht darin, dass mit einem erhöhten Anteil an Sisalsäure die Halbwertzeit des EPO im Plasma zunimmt, da nur ein nicht-sialysiertes EPO an dem am EPO-Abbau beteiligten Galactose-Rezeptor der Leber binden kann. Dieses EPO-Derivat - bekannt unter der Abkürzung NESP *(Novel Erythropoiesis Stimulating Protein* oder Darbepoetin) - weist zwar im Vergleich zum rekombinanten EPO eine an fünf Positionen veränderte Aminosäuresequenz auf. Es entspricht aber in seiner Wirkung auf die Stimulation der Erythrozytenbildung im Wesentlichen dem nativen oder rekombinanten EPO (Fisher, J.W.: Erythropoietin: Physiology and Pharmacology Update. Erythropoietin 2003, 1-14). Die EP 0 640 619 B1 wird hinsichtlich der Struktur und Herstellung des NESP vollumfänglich in Bezug genommen. Das NESP kann zur weiteren Verlängerung der *in vivo* Halbwertzeit in vorteilhafter Weise noch mit Polyethylenglykol (PEG) chemisch konjugiert werden, ohne dass damit eine Veränderung der biologischen Aktivität verbunden wäre. Ein derart modifiziertes NESP ist aus der WO 01/76640 bekannt, die hinsichtlich der Struktur und Bereitstellung dieses EPO-Derivats vollumfänglich in Bezug genommen wird. Aus der WO 02/49673 A2 und WO 01/02017 A2 sind ebenso pegylierte Derivate des EPO bekannt, die neben der verlängerten Plasma-Halbwertzeit auch eine höhere klinische Wirksamkeit zeigen. Dazu werden beispielsweise durch gezielte Mutagenese 1 bis 6 zusätzliche Glykosylierungsstellen in die EPO-Aminosäuresequenz eingebracht. Auch dieses Derivat kann eingesetzt werden.

Erfindungsgemäß kann EPO sowohl in seiner humanen als auch in seiner nicht-humanen Form eingesetzt werden.

Die einzelnen Konzentrationen der Wachstumsfaktoren können in Lösung bei ca. 1 bis ca. 100 ng/ml, vorzugsweise bei ca. 10 bis ca. 50 ng/ml, insbesondere bei ca. 10 bis ca. 20 ng/ml liegen. Bei lokalen Beschichtungen (topischer Verabreichung) können die Konzentrationen der Wachstumsfaktoren jedoch auch ein Vielfaches davon betragen.

Die hämatopoietischen Wachstumsfaktoren können vorteilhafterweise auch für die Kultivierung von Gewebekulturen *in vitro* eingesetzt werden. Dazu werden die Zellen in für das Gewebe besonders geeigneten Vorrichtungen und Verfahren kultiviert, beispielsweise auf einem Gitter mit einer schneidenden Maschenstruktur von 500 µm Größe.

Insbesondere in vitro sind Kombinationen von EPO mit GH vorteilhaft. Insbesondere können berührungslose, automatisch oder manuell gesteuerte Pumpsysteme eingesetzt werden, die z. B. aus Kolbenpumpen bestehen oder magnetisch bzw. durch Druckluftkompression von Schläuchen erzeugte, gerichtete Ströme erzeugen. In Anwesenheit von Endothelzellen kann es durch den Scherstress in einem perfundierten Bioreaktor zu einer spontanen Konfluenz der Endothelzellen auf den Oberflächen der Aggregate kommen, was für die weitere Verwendung vorteilhaft sein kann.

Für die Einkapselung eignen sich dem Fachmann bekannte, geeignete Materialien, in die beispielsweise strukturierte Formen oder Räume integriert werden, die eine *in situ* Wachstumsstruktur bzw. Vergrößerung ermöglichen. Alternativ kann auf die Kapsel verzichtet werden und beispielsweise in Gegenwart von Endothelzellen eine Endothelialisierung und somit eine optimale Hämokompatibilität erreicht werden.

Die Gabe von EPO führt entweder alleine oder bei größeren strukturellen Defekte auch in Verbindung mit Scaffold-Materialien zu einem biologischen Gewebeersatz. Diese Trägermaterialien können in vitro bzw. extrakorporal vorbesiedelt sein, biologisch modellierbar (biodegradabel) sein und sind mikro- und makrostrukturell und biochemisch im Idealfall der zu ersetzenden Struktur möglichst ähnlich. Die biochemische Nähe bzw. Identität beinhaltet eine Rekonstruktion der in vivo Zusammensetzung durch Kollagene und Matrixproteine (Elastin, Fibronektin und alle Matrixkomponenten des Körpers in gewebespezifischer Prägung wie bekannt).

Stammzellen können aus den verschiedenen im Körper des Patienten vorhandenen Quelle erhalten werden: Knochenmark, peripheres Blut, Fettgewebe, dem Gewebe selbst, Nabelschnurblut- bzw. Gewebe. Allogene Stammzellen können entsprechend gewonnen werden, sind aber mit immunologischen Nachteilen behaftet. Embryonale Nichtmenschliche embryonale Zellen können verwendet werden, sind aber mit den entsprechenden Nachteilen behaftet.

In einer besonders bevorzugten Ausführungsform kann der strukturierte Wachstumsprozess der kultivierten Zellen durch eine mit den Wachstumsfaktoren beschichteten Matrix induziert werden. Dazu kann die Matrix mit einem oder mehreren der genannten Wachstumsfaktoren sequentiell behandelt werden.

Sofern es sich um eine biologische Matrix handelt, ist es üblicherweise ein Implantat (Stent, Patch oder Katheter), ein Transplantat (z. B. Hauttransplantat), ein Trägermaterial zum Wachstum von Zellen, z.B. ein sogenanntes Slow-Release Material (z.B. ein Hydrogel auf der Basis von Fibrin und/oder Polymere, wie z. B. Polylaktid oder Polyhydroxyalkanoat, und/oder Alginate), ein Knochenersatzmaterial (z.B. Tricalciumphosphat), ein allogenes, autologes oder xenogenes azellularisiertes oder nicht-azellularisiertes Gewebe (z.B. Herzklappe, Venenklappe, Arterienklappe, Haut, Gefäß, Aorta, Sehne, Comea, Knorpel, Knochen, Trachea, Nerv, Miniskus, Diskus intervertebralis, Ureteren, Urethra oder Blase (siehe z. B. EP 0 989 867 oder EP 1 172 120)), oder eine Matrix (z.B. eine Laminin-, Collagen IV- und/oder Matrigel-Matrix) oder vorzugsweise ein Feeder-Layer, wie z.B. Collagen I-, 3T3- und/oder MRC-5-Feeder Layer, oder ein Collagenvlies. Die biologische Matrix wird vorteilhafterweise mit gewebespezifischen Zellen, Vorläuferzellen, Knochenmarkzellen, peripheres Blut, Fettgewebe und/oder Fasergewebe, z.B. mit adulten Vorläuferzellen aus dem Knochenmark, vorbesiedelt. Die Vorbesiedelung führt dazu, dass der Regenerationsprozess bereits *in vitro* einsetzt damit nach der Implantation der Matrix in den Körper die Regenerationszeit *in vivo* verkürzt ist. Die verwendeten Matrices können noch zuvor aktiviert werden. Die Aktivierung der biologischen Matrix oder Trägerstruktur kann beispielsweise mittels Plasmaionisation, z.B. unter Verwendung von Wasserstoffperoxid, oder mittels Laseraktivierung erfolgen. Alternativ kann eine Beschichtung mit einer biodegradablen (Bio)polymerschicht, die den oder die genannten Wachstumsfaktor(en) enthält, vorgenommen werden. Hierzu eignen sich beispielsweise Fibrin, Plasma, Blut, Collagen und/oder Polylaktide.

Das Verfahren eignet sich insbesondere für adulte Zellen, d. h. primär differenzierte Zellen, die keinen embryonalen oder fötalen Phänotyp mehr besitzen. Es eignet sich insbesondere für humane adulte Zellen, beispielsweise für adulte Progenitorzellen, gewebespezifische Zellen, vorzugsweise Osteoblasten, Fibroblasten, Hepatozyten und/oder glatte Muskelzellen.

Neben einer Beendigung bzw. Reduzierung der Zufuhr der beschriebenen Wachstumsfaktoren zu der Kultur eignen sich zur Terminierung des Wachstumsprozesses auch Somatostatin und/oder TGF beta und/oder Prostaglandine. Es ist besonders vorteilhaft, dass Verfahren lokal *in vivo* einzusetzen. Dazu können die Wachstumsfaktoren z.B. auf die Resektionsfläche eines Organs (z. B. einer Leber) aufgebracht werden. Sie können topisch oder aber über mit Hilfe eines Katheters lokal oder systemisch appliziert werden. Ebenso ist es möglich, die Wachstumsfaktoren (z.B. zur Förderung der Knorpelregeneration) unmittelbar in das betroffene Gewebe oder Gelenk zu injizieren. Damit können die Wachstumsfaktoren über die Synovialflüssigkeit direkt auf die Bildung einer neuen Knorpelstruktur wirken.

In einer besonderen Ausführungsform können zusätzlich zu den hämatopoietischen Wachstumsfaktoren einer oder mehrerer der folgenden Wachstumsfaktoren verabreicht werden: "Transforming Growth Factor beta" (TGF beta), Prostaglandine, Granulozyten (Makrophagen)-stimulierender Faktor (G(M)-CSF), "Growth Hormone Releasing Hormone" (GHRH), "Thyrotropin-releasing Homone" (TRH), "Gonadotropin-releasing Hormone" (GnRH), "Corticotropin-releasing Hormone" (CRH), Dopamin, "Antidiuretic Hormon" (ADH), Oxytocin, Prolactin, Adrenocorticotropin, beta-Celltropin, Lutrotropin und/oder Vasopressin verwendet bzw. zusätzlich ein oder mehrere Nervenregenerationsfaktoren, vorzugsweise "nerve growth factor" (NGF) und/oder ein oder mehrere Gefäßregenerationsfaktoren, vorzugsweise "Vascular Endothelial Growth Factor" (VEGF) und/oder "Plateled Derived Growth Factor" (PDGF).

Die hämatopoietischen Wachstumsfaktoren parenteral als injizierbare Mikrosphären, erodierbare Partikel, Polymerverbindungen (mit Polypeptid, Polyglycolsäure), Liposomen und kleine Partikel appliziert werden. Injizierbare oder implantierbare Drug Delivery -Geräte sind ebenso möglich. Die Stoffe können auch inhaliert werden, subkutan, intramuskulär gespritzt werden oder - für die topische Applikation als kutanes Pflaster gegeben werden. Begleitend können Substanzen mit den Pflastern vermischt werden, die eine lokale Hyperämie erreichen und dadurch die Hautpermeabilität erhöhen (z.B. Bienengift). Hyperionische Strukturen mit und ohne direkte EPO Kopplung bzw. Beschichtung der Salze können durch die Hautbarrieren besser transportiert werden und können mit bevorzugt positiv ionisierenden Strukturen verbunden werden. Auch negative Ionisation ist möglich. Die Faktoren können mit Mandelöl bzw. Jojobaöl zur Transportverbesserung durch Schleimhäute im Darmbereich bzw. der Haut verwendet werden. Eine Verbindung mit Polyethylenglykol (PEG) ist möglich, um Transportbarrieren (Mucosa im Mund, Magen, Darm; Schleimhäute, Hornhaut) zu überwinden. Es ist bekannt, dass aus Proteinen Mischpräparationen vorbereitet werden können. Suspensionen, Gelimulsionen, Feststoffverbindungen, dehydrierte oder lyophilisierte Puder sind möglich. Die Wachstumsfaktoren können auf Partikeln absorbiert werden oder enkapsuliert werden.

Es kann besonders vorteilhaft sein, Stammzellen (Progenitoren im eigentlichen Sinne) gemeinsam mit EPO zur Geweberegeneration einzusetzen, um damit das Recruitment der Gewebezellen aus den 4 Grundgewebearten für den Regenerationsprozess deutlich zu beschleunigen. EPO sowie die anderen genannten Faktoren können gemischt mit Stammzellen und z.B. Fibrinkleber als Trägermatrix appliziert werden. Bei Bedarf kann die Trägermatrix weggelassen werden bzw. durch eine stärker vorstrukturierte und geformte Trägermatrix ersetzt werden. Die Faktoren können auch ohne jegliche biologische Trägermatrix z.B nur in wässriger Suspension systemisch oder topisch verabreicht werden.

Die Gabe von EPO verbessert diese Geweberegeneration durch gewebespezifische Prägung der Stammzellen und Differenzierung im Anschluss an eine Vermehrung und Integration und koordiniert das Wachstum in Bezug auf die Grundgewebearten.

### EPO wird wie folgt eingesetzt:

Reparative Prozesse nach Verbrennungen, Trauma, Verbrühungen, mechanischen Verletzungen oder Entzündungen im Bereich der Haut sind sehr vielfältig. Durch Gabe von EPO kann mit und ohne strukturelle Ersatzstrukturen eine Regeneration erreicht werden.

Insbesondere bei chronischen Erkrankungen, Durchblutungsstörungen, diabetischen Ulzera, Phänomenen auch immunologischer Natur kann EPO hier modellierend eingreifen. Histologisch gesehen kommt es zu einer Wiederausbildung einer normalen Epidermis, Dermis und einer Subkutis, die eine entsprechende Vaskularisation mit Makrostrukturen erreicht. An den Handflächen und Fußsohlen kommt es zur Ausbildung des stratum basale, stratum granulosum, stratum lucidum, stratum coneum. An der Dermis und der Außenseite kommt es zur Ausbildung von Dermispapillen. An der Epidermis (innere Oberfläche) werden die entsprechenden Epidermisvertiefungen wieder ausgebildet. Zusätzlich werden Anhangsgebilde und deren Ausbildungen aus Progenitoren mitunterstützt. Ein Problem beim bisherigen Tissue Engineering war die Verbindung struktureller Reparatur mit der Ausbildung von Hautanhangsgebilden im Bereich der Haut.

Durch Gabe von EPO können Interaktionsprozesse im Bereich des Melanozytensystems des Menschen erreicht werden. Physiologische Rückkopplungsprozesse zwischen Augen, Hirnhaut und der Ausbildung der Pigmentierung werden berücksichtigt, da es sich hier nicht um isolierte Zellverbindungen, sondern organtypische Regenerationsprodukte handelt. Reparative Prozesse des dermalen Anteiles beinhalten muskuläre Strukturen (musculus arector pili), taktile Strukturen, äußere Wurzelscheide der Haare und innere Wurzelscheide und auch die Haarzwiebelstruktur. Diese ragt unter Umständen in das Fettgewebe hinein. Bei den Haaren und Haarfollikeln kommt es zur Ausbildung von bindegewebigen Wurzelscheiden, die eine wichtige Funktionsstruktur in der Regeneration beinhaltet. Daraus bilden sich Glashaut, äußere Wurzelscheide, innere Wurzelscheide und das letztendliche Haar. Ähnliche Prozesse können im Bereich der Nagelregeneration unterstützt und herbeigeführt werden. Innerhalb der Gewebestrukturen werden Schweißdrüsen mit ausgebildet. In den Hautstrukturen werden perifollikuläre Netzstrukturen, Netze, subkapilläre Arteriennetze, termale Venennetze wieder ausgebildet.

Bei einer Behandlung von 8 brandverletzten Patienten heilten in einem konkreten Fallbeispiel bei EPO Gabe die Donorstelle des Hauttransplantates um 50% schneller ab. Tief zweitgradige (Grad 2B) Verbrennungswunden im Gesicht heilten ohne Narbenbildung ab, wenn EPO den Patienten gegeben wurde. Ohne EPO Gabe heilten derartige Wunden mit Narbenbildung ab.

Folgende Gegenstände sind auch hier beschrieben:
- Verwendung von hämatopoietischen Wachstumsfaktoren, insbesondere von Erythropoietin (EPO) oder deren Derivate, Analoga oder Teile zur Herstellung eines Medikaments zur Förderung der strukturellen Regeneration von insbesondere traumatisiertem Gewebe, insbesondere bei Verbrennungen und Verbrühungen, wobei vorzugsweise die Rezeptor-Binde-Domäne der Wachstumsfaktoren bzw. deren Derivate oder Analoga beteiligt ist.
- Eine entsprechende Verwendung unter zusätzlicher Beteiligung der folgenden Faktoren: Somatostatin, "Ciliary Neurotropic Factor" (CNTF), "Transforming Growth Factor beta" (TGF beta), Prostaglandine, Granulozyten-Makrophagen-stimulierender Faktor (GM-CSF), Granulozyten-stimulierender Faktor (G-CSF), Growth Hormone Releasing Hormone (GHRH), Dopamin, Antidiuretic Hormon (ADH), Oxytocin, Prolactin, Adrenocorticotropin, beta-Celltropin, Lutrotropin, Vasopressin, Nervenregenerationsfaktoren, vorzugsweise Nerve Growth Factor (NGF), Gefäßregenerationsfaktoren, vorzugsweise Vascular Endothelial Growth Factor (VEGF) oder Plateled Derived Growth Factor (PDGF).

- Eine entsprechende Verwendung bei Anwesenheit von Endothelzellen.
- Eine analoge Verwendung bei der die Regeneration des Gewebes lokal gelenkt wird.
- Eine entsprechende Verwendung bei welcher das EPO aufweisende Medikament topisch und / oder systemisch verabreicht wird.
- Eine bisher beschriebene Verwendung von EPO bei welcher der Wachstumsprozess durch eine Trägerstruktur unterstützt wird, wobei die Trägerstruktur mit dem Wachstumsfaktor behandelt wird.
- Eine bisher beschriebene Verwendung von EPO bei welcher als Trägerstruktur ein Implantat, ein Transplantat oder ein Trägermaterial zum Wachstum von Zellen verwendet wird.
- Eine bisher beschriebene Verwendung von EPO bei welcher die Trägerstruktur mit Zellen, vorzugsweise gewebespezifischen Zellen, Vorläuferzellen, Knochenmarkszellen, peripherem Blut, Fettgewebe oder Fasergewebe vorbesiedelt oder für die *in vivo* Besiedelung bzw. das induktive Remodelling *in vitro* vorbereitet wurde.
- Eine bisher beschriebene Verwendung von EPO, wobei die Trägerstruktur mit mindestens einem der Wachstumsfaktoren, dessen Derivate, Analoga oder Teile beschichtet wird, wobei sie vorzugsweise *in vitro* mit Zellen, vorzugsweise gewebespezifischen Zellen, Vorläuferzellen, Knochenmarkzellen, peripherem Blut, oder Fasergewebe vorbesiedelt wird.
- Eine bisher beschriebene Verwendung von EPO, bei welcher der Wachstumsprozess durch Stammzellengabe aus dem Knochenmark, Blut, Gewebe, Fettgewebe, Nabelschnurgewebe bzw. Blut unterstützt wird.

## Patentansprüche

1. Erythropoietin (EPO) zur Anwendung bei der Wundheilung ohne Narbenbildung nach Brandverletzungen unter Verwendung eines Hauttransplantats.

2. Erythropoietin (EPO) zur systemischen oder topischen Anwendung bei der Regeneration von durch Verbrennungen oder Verbrühungen traumatisiertem Gewebe unter Verwendung eins Transplantates, Implantates oder eines Trägermaterials.

3. Erythropoietin (EPO) zur topischen Anwendung nach Anspruch 2.

4. Erythropoietin (EPO) zur Anwendung nach Anspruch 2 oder 3 bei Brandverletzungen unter Verwendung eines Hauttransplantats.

## Claims

1. Erythropoietin (EPO) for use in wound healing without scarring after burn injuries using a skin graft.

2. Erythropoietin (EPO) for systemic or topical use in the regeneration of tissue traumatised by burns or scalds using a graft, implant or support material.

3. Erythropoietin (EPO) for topical use according to Claim 2.

4. Erythropoietin (EPO) for use according to Claim 2 or 3 in burn injuries using a skin graft.

## Revendications

1. Erythropoïétine (EPO) pour une utilisation au niveau de la guérison de plaies sans cicatrice après des lésions par brûlures en utilisant une greffe de peau.

2. Erythropoïétine (EPO) pour une utilisation systémique ou topique au niveau de la régénération d'un tissu traumatisé par des brûlures ou des échaudures en utilisant une greffe, un implant ou un matériau de support.

3. Erythropoïétine (EPO) pour une utilisation topique selon la revendication 2.

4. Erythropoïétine (EPO) pour une utilisation selon la revendication 2 ou 3 au niveau de lésions par brûlures en utilisant une greffe de peau.
